# EUROPÄISCHE PATENTSCHRIFT

(11) **EP 0 876 316 B1**
(45) Veröffentlichungstag und Bekanntmachung des Hinweises auf die Patenterteilung: **05.04.2000**
(21) Anmeldenummer: 96938170.6
(22) Anmeldetag: 11.11.1996
(51) Int. Cl.: C07C 29/38, C07C 31/22, C07C 35/08, C07C 67/44, C07C 29/09

(54) **VERFAHREN ZUR HERSTELLUNG VON POLYALKOHOLEN**
PROCESS FOR THE PREPARATION OF POLYALCOHOLS
PROCEDE DE PREPARATION DE POLYALCOOLS

(30) Priorität: 10.11.1995 DE 19542036
(43) Veröffentlichungstag der Anmeldung: 11.11.1998
(73) Patentinhaber: BASF AKTIENGESELLSCHAFT, 67056 Ludwigshafen (DE)
(72) Erfinder: KRATZ, Detlef, D-69121 Heidelberg (DE); STAMMER, Achim, D-67251 Freinsheim (DE); WITZEL, Tom, D-67069 Ludwigshafen (DE); BRUDERMÜLLER, Martin, D-68239 Mannheim (DE)
(74) Vertreter: Isenbruck, Günter, Dr.
(86) Internationale Anmeldenummer: EP9604922
(87) Internationale Veröffentlichungsnummer: WO9717313

(56) Entgegenhaltungen:
- EP-A- 0 142 090
- EP-A- 0 289 921
- WO-A-94/07831
- DE-A- 2 507 461
- DE-A- 2 813 201
- JOURNAL OF GENERAL CHEMISTRY USSR, Bd. 57, Nr. 7, Pt.2, Juli 1987, NEW YORK, US, Seiten 1411-1413, XP002025173 R. NURBERDIEV, ET AL.: "Homolytic reactions of formates of alcohols and glycols"

## Beschreibung

Die Erfindung betrifft ein Verfahren zur Herstellung von Polyalkoholen, d.h. Polymethylolalkanen und Polymethylolcycloalkanen, durch Umsetzung von Alkanalen oder Ketonen (welche cyclische Ketone umfassen) mit Formaldehyd in Gegenwart eines tertiären Amins.

Es ist bekannt, daß Alkanale mit überschüssigen Mengen Formaldehyd unter Zusatz stöchiometrischer Mengen einer anorganischen Base (z.B. Natronlauge, Calciumhydroxid, Calciumoxid, Bariumhydroxid) in wäßriger Lösung zu Di-, Tri- und im Falle von Acetaldehyd Tetramethylolalkanen umgesetzt werden können. Der Reaktionsverlauf vollzieht sich in mehreren Teilschritten. Nach ein- oder zweifacher, im Falle von Acetaldehyd auch dreifacher Aldolreaktion von Formaldehyd und dem Alkanal zu dem entsprechenden Mono-, Di- oder im Falle von Acetaldehyd Trimethylolalkanal werden in einer gekreuzten Cannizzaro-Reaktion mit Formaldehyd unter Wirkung der anorganischen Base der Polyalkohol und ein Alkali- oder Erdalkaliformiat gebildet.

Die bei dieser Vorgehensweise anfallenden Mengen an anorganischem Formiat stören die Isolierung des Polyalkohols und müssen vor der Reinigung quantitativ abgetrennt werden. Für die Trennung gibt es mehrere Methoden, von denen die Extraktion des Polyalkohols, die Elektrodialyse oder die Fällung des anorganischen Salzes durch Lösungsmittelwechsel die gebräuchlichsten sind. Nachteilig ist der hohe Aufwand für diese Verfahren, zudem bleibt ein mit dem Extraktionsmittel belasteter wäßriger Rückstand des anorganischen Salzes zurück, der zu erheblicher Umweltbelastung führt.

Verschiedene andere Verfahren zur Lösung dieses Problems bei der Herstellung von Polyalkoholen sind vorgeschlagen worden.

In DE-OS 25 07 461 wird ein Verfahren für die Herstellung von 2,2-Dimethylolalkanalen beschrieben. Diese werden durch Umsetzung von Aldehyden der Formel 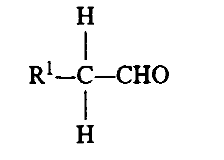 worin R¹ ein aliphatischer Rest ist, mit Formaldehyd in Gegenwart von tertiären verzweigten Alkylaminen erhalten. Die nach diesem Verfahren hergestellten 2,2-Dimethylolalkanale sind wertvolle Zwischenprodukte für die Herstellung von Trimethylolalkanen durch Hydrierung. Eine Cannizzaro-Reaktion wird vermieden. Beispielsweise ergibt die Reaktion von n-Butyraldehyd mit Formaldehyd unter katalytischer Wirkung von verzweigtem tertiärem Amin Dimethylolbutanal. Dieses wird destilliert und kann anschließend zu Trimethylolpropan (TMP) hydriert werden, allerdings sind die hierbei erhaltenen Ausbeuten unbefriedigend.

Die DE-OS 28 13 201 beschreibt ebenfalls ein Verfahren für die Herstellung von 2,2-Dimethylolalkanalen, wobei Aldehyde mit Formaldehyd im Molverhältnis 1:5 - 1:30 in Gegenwart von Hydroxiden und/oder Carbonaten der Alkali- und /oder Erdalkalimetalle und /oder tertiären Aminen umgesetzt werden. Es kann eine Hydrierung zu Trimethylolalkanen folgen. Vor der Reduzierung zum Trimethylolalkan sollten das gegebenenfalls eingesetzte Amin, der im Überschuß eingesetzte Formaldehyd sowie nicht umgesetzter Aldehyd abgetrennt werden. Es muß mit einem hohen Formaldehydüberschup zur Erzielung besserer Ausbeuten gearbeitet werden.

Für eine wirtschaftliche Herstellung von Trimethylolpropan ist dieses Verfahren wenig geeignet, da der große Formaldehydüberschuß vor der katalytischen Hydrierung abgetrennt werden muß.

In der EP-A-0 142 090 ist ein Verfahren zur Herstellung von Trimethylolalkanen durch Umsetzung von 1 Mol n-Alkanal mit 2,2-4,5 Molen Formaldehyd und 0,6-3 Molen Trialkylamin in wäßriger Lösung und nachfolgender Hydrierung offenbart. Hierbei wird das Reaktionsgemisch nach oder vor der Hydrierung destillativ aufgearbeitet.

Bei der Umsetzung wird entstehende Ameisensäure als Trialkylammoniumformiat gebunden und destillativ entfernt. Die destillative Trennung ist großtechnisch schwer durchzuführen, wenn sehr reine Produkte erhalten werden sollen.

Die EP-A-0 289 921 beschreibt ein Verfahren, bei dem wie bei dem in EP-A-0 142 090 beschriebenen Verfahren das Synthesegemisch hydriert wird. Gemäß der Ausführungsform (a) des in EP-A-0 289 921 beschriebenen Verfahrens erhitzt man bei der Herstellung von Trimethylolalkanen durch Umsetzung von n-Alkanalen mit 2,2-4,5 Mol Formaldehyd in wäßriger Lösung in Gegenwart von 0,6-3 Mol Trialkylamin, jeweils bezogen auf 1 Mol Alkanal, und anschließende Hydrierung, das rohe Umsetzungsgemisch auf Temperaturen von 100-200°C, wobei man das im Reaktionsgemisch vorhandene Wasser weitgehend, gegebenenfalls zusammen mit überschüssigem, freiem Trialkylamin destillativ abtrennt und das vorliegende Trialkylammoniumformiat mit dem Trimethylolalkan unter Bildung von Trimethylolalkanformiat, und Trialkylamin umsetzt, das Trialkylamin abdestilliert und das Trimethylolalkanformiat mit Methanol gegebenenfalls in Gegenwart katalytischer Mengen an Alkali- oder Erdalkalialkoholaten zu Trimethylolalkan und Methylformiat umestert. Das Methylformiat wird entfernt.

Nachteilig bei diesem Verfahren ist, daß zur Erzielung hoher Ausbeuten eine Hydrierung des rohen Synthesegemisches erforderlich ist und der im Überschuß eingesetzte Formaldehyd bei der Hydrierung in Form von Methanol verloren geht, und dadurch die wirtschaftliche Umsetzung des Verfahrens erheblich eingeschränkt ist.

Gemäß der Verfahrensvariante (b) des in der EP-A-289 921 beschriebenen Verfahrens wird nach katalytischer Hydrierung das Synthesegemisch weitgehend entwässert, wobei gegebenenfalls überschüssiges freies Trialkylamin dabei abgetrennt wird. Das in Form von Trialkylammoniumformiat vorliegende Trialkylamin wird freigesetzt, indem das verbleibende Gemisch mit Methanol versetzt und auf Temperaturen von 100-200°C unter Bildung von Methylformiat und Trialkylamin erhitzt wird. Die Reaktionsprodukte werden in an sich bekannter Weise isoliert.

Wie bei der Verfahrensvariante (a) ist auch bei der Verfahrensvariante (b) zur Erreichung befriedigender Ausbeuten eine Hydrierung des Syntheseaustrags notwendig und damit der Verlust des überschüssigen Formaldehyds bedingt.

Die bisher beschriebenen Verfahren beziehen sich zudem lediglich auf die Herstellung von Dimethylolalkanen und/oder Trimethylolalkanen.

WO 94/07831 betrifft die Herstellung von Trimethylolpropan. Dabei wird gemäß einer Ausführungsform n-Butyraldehyd mit Paraformaldehyd in Abwesenheit einer wäßrigen Lösung in Gegenwart von katalytischen Mengen Triethylamin umgesetzt. Bei der ersten Umsetzung wird Formaldehyd gemäß einer Aldolreaktion an den n-Butyraldehyd angelagert, so daß die Carbonylfunktion des Butyraldehyds im Molekül verbleibt. In einem nachfolgenden Reaktionsschritt wird das erhaltene Produkt in Gegenwart von Kupferchromit mit Wasserstoff zu Trimethylolpropan hydriert. Bei der Aldolreaktion wird Triethylamin in einer Menge von 0,04 bis 0,05 Äquivalenten eingesetzt.

Erwünscht ist jedoch ein Verfahren, das auch die Herstellung von Tetra- und höheren Polymethylolalkanen und Polymethylolcycloalkanen, bei dem nicht äquimolare Mengen an anorganischem Formiatsalz gebildet werden, ermöglicht, und zugleich in wenigen Stufen wirtschaftlich durchführbar ist.

Aufgabe der vorliegenden Erfindung ist es, ein Verfahren zur Herstellung von Polyalkoholen, welche Polymethylolalkane und Polymethylolcycloalkane umfassen, zur Verfügung zu stellen.

Dabei soll das Verfahren die Herstellung von sehr reinen Polyalkoholen auf einfache und in wirtschaftlicher Hinsicht effiziente Weise ermöglichen. Ein weiteres Ziel der Erfindung ist es, eine hohe Ausbeute zu erzielen. Außerdem soll erfindungsgemäß ein Verfahren zur Herstellung von Polyalkoholen zur Verfügung gestellt werden, welches mit möglichst wenig Stufen und ohne Hydrierungen auskommt, wenig Abfallprodukte, insbesondere keine Alkaliformiate, erzeugt und eingesetzte Materialien vorzugsweise in mehreren Stufen nutzt.

Gemäß einer ersten Ausführungsform der Erfindung wird ein Verfahren zur Herstellung eines Formiate enthaltenden Polyalkoholprodukts zur Verfügung gestellt. Bei diesem Verfahren wird Alkanal oder Keton mit Formaldehyd in wäßriger Lösung in Gegenwart von tertiärem Amin umgesetzt, wobei diese Umsetzung erfindungsgemäß temperaturkaskadiert erfolgt. Dabei wird die Umsetzung temperaturkaskadiert mit steigender Temperatur, vorzugsweise mit einer zwei- bzw. dreistufigen Temperaturkaskade durchgeführt. Besonders bevorzugt ist es, die temperaturkaskadierte Umsetzung in einem Temperaturbereich von 20-90°C und während einer Dauer von 0,5-24 Stunden, insbesondere 1-6 Stunden, durchzuführen. "Temperaturkaskadiert" bedeutet dabei eine sequentielle stufenweise Umsetzung auf unterschiedlichen Temperaturstufen, wobei die Temperatur von Stufe zu Stufe zunimmt. Die Umsetzung erfolgt somit nacheinander in Stufen mit zunehmender Temperatur. Bezogen auf 1 Mol Alkanal oder Keton werden 0,6 bis 5 Mol tertiäres Amin eingesetzt. Das Verfahren wird ohne Hydrierung des Produktgemisches durchgeführt.

Diese Umsetzung führt in hohen Ausbeuten zu Polyalkanolprodukten.

Weiterhin wird die obige Aufgabe gelöst durch ein Gesamtverfahren zur Herstellung von Polyalkoholen. Dieses Verfahren umfaßt die folgenden Stufen:
(a) Umsetzung eines Alkanals oder Ketons mit Formaldehyd in wäßriger Lösung in Gegenwart eines tertiären Amins zu einem Formiate enthaltenden Polyalkoholproduktgemisch,
(b) Abtrennung von Wasser, überschüssigem tertiärem Amin, überschüssigem Formaldehyd,
(c) Erhitzen des verbliebenen Gemisches von (b) unter Abtrennung von weiterem Formaldehyd und tertiärem Amin und Bildung von Polyalkoholformiaten,
(d) Überführung von abgetrenntem tertiärem Amin aus der Stufe (b) und/oder der Stufe (c) in die Synthesestufe (a) und/oder in die folgende Umesterungsstufe (e),
(e) Umesterung von erhaltenen Polyalkoholformiaten aus der Stufe (c) mit einem Alkohol der Formel ROH in Gegenwart eines Umesterungskatalysators zu Polyalkoholen und Formiaten der Formel 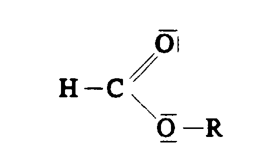 wobei R einen Kohlenwasserstoffrest, vorzugsweise Alkylrest mit 1-6, besonders bevorzugt 1-2 C-Atmen, bedeutet,
(f) Gewinnung von Polyalkoholen.

Das tertiäre Amin wird dabei in die Umsetzung zurückgeführt, was die Wirtschaftlichkeit des Verfahren erhöht. Es können auch alle in Stufen (b) und (c) entfernten Stoffe, gegebenenfalls nach Abtrennung von Leicht- und Mittelsiedern, zurückgeführt werden.

Die Umsetzung von Alkanal oder Keton mit Formaldehyd in Gegenwart von tertiärem Amin erfolgt diskontinuierlich oder kontinuierlich. Die kontinuierliche Arbeitsweise ist bevorzugt.

Das Produktgemisch in Stufe (a) enthält dabei vornehmlich Polyalkanol, Polyalkoholhalbformale, bereits gebildete Polyalkoholformiate und Trialkylammoniumformiat. Aufgrund der weitgehenden Umsetzung zu Polyalkoholen kann auf eine Hydrierung verzichtet werden.

In der Stufe (a) kann zudem Methanol durch Cannizzaro-Reaktion von Formaldehyd zu Ameisensäure und Methanol gebildet werden. Dieses wird bevorzugterweise in der Stufe (b) zusammen mit Wasser, überschüssigem tertiärem Amin und überschüssigem Formaldehyd von der übrigen Reaktionsmischung abgetrennt. Diese Abtrennung geschieht insbesondere mittels Destillation.

Das Verfahren gemäß der Erfindung wird vorzugsweise so durchgeführt, daß Polyalkohole ohne Hydrierung hergestellt werden.

Weiterhin zeichnet sich das erfindungsgemäße Verfahren in einer besonderen Variante dadurch aus, daß tertiäres Amin, Wasser und nicht umgesetzter Formaldehyd im Kreislauf geführt werden und das Verfahren sich somit in wirtschaftlicher Hinsicht sehr effizient gestaltet.

Gemäß einer Variante des Verfahrens wird die Umesterung der Formiate mit einem leichtsiedenden Alkohol in Gegenwart von tertiärem Amin durchgeführt, wobei als tertiäres Amin insbesondere das gleiche Amin wie in der Synthesestufe verwendet werden kann.

Die Herstellung des rohen Synthesegemisches (Stufe (a) beispielsweise Herstellung des Methylolprodukts) erfolgt diskontinierlich oder kontinuierlich, vorzugsweise kontinuierlich, mit Unterdruck, Überdruck oder drucklos, vorzugsweise drucklos. Die Umsetzung erfolgt im allgemeinen bei Temperaturen bis 200°C, vorzugsweise bei 10-120°C, insbesondere zwischen 30 und 80°C. Bei kontinuierlicher Durchführung wird z.B. eine Reaktorkaskade aus zwei bis fünf, vorzugsweise drei hintereinander geschalteten Rührkesseln verwendet, die durch Kühlung oder Beheizung verschiedene Temperaturen aufweisen können. Die Temperaturen der einzelnen Kessel liegen zwischen 10 und 150°C.

Überraschenderweise werden durch Temperaturstaffelung T (Kessel n) > T (Kessel n-1), wobei n vorzugsweise 2-4 ist, z.B. in einer dreistufigen Reaktorkaskade mit einem Temperaturgradienten von 20-40°C/40-70°C/70-90°C, vorzugsweise etwa 40°C/60°C/80°C, erhöhte Rohausbeuten an Polyalkoholen erzielt und die Bildung von Nebenprodukten (z.B. dimere Ether des Polyalkohols, Formaldehyd-bis(polyalkohol)-acetale, cyclische Acetale des Polyalkohols als auch Dehydratisierungsprodukte) erheblich vermindert. Überschüssiger Formaldehyd wirkt sich nicht störend aus, er liegt als Halbformal in reversiblem Gleichgewicht an die Alkoholfunktionen des Polyalkohols gebunden vor.

In vorteilhafter Weise können bei dem erfindungsgemäßen Verfahren sich bildende dimere Ether des Polyalkohols sowie gegebenenfalls andere höhere oder abgewandelte Polyalkoholprodukte als Wertprodukt gewonnen werden. Dieser Vorteil wird dadurch erzielt, daß das tertiäre Amin zur Umsetzung bzw. Umesterung eingesetzt wird und nicht beispielsweise Natriumhydroxid. Bei Verwendung von solchen Katalysatoren bilden sich Salze, so daß die oben erwähnten Wertprodukte jedenfalls nicht wirtschaftlich gewonnen werden können.

Die Reaktionsdauer kann zwischen 0,5 und 24 Stunden betragen, bevorzugt werden Zeiten von 1-6 Stunden.

Formaldehyd wird in der technisch gebräuchlichen Form als 10-50%ige wäßrige Lösung eingesetzt, der Wassergehalt der Reaktionsmischung liegt zwischen 40 und 85%, vorzugsweise zwischen 60 und 75%, insbesondere bei 68%.

Als Alkanale werden vorzugsweise solche mit 2-12, insbesondere 2-8 C-Atomen verwendet. Beispiele für besonders geeignete Alkanale sind Acetaldehyd, n-Propanal, n-Butanal, n-Pentanal, 3-Methylbutanal, n-Hexanal, 3-Methylpentanal, 2-Ethylhexanal, n-Heptanal.

Als Ketone werden vorzugsweise aliphatische Ketone, bevorzugt solche mit 3-18, insbesondere 3-8 C-Atomen verwendet. Bevorzugt sind cyclische Ketone mit 4-8, insbesondere mit 6 C-Atomen. Besonders bevorzugt wird Cyclohexanon verwendet. Die Aldehyd- oder Ketofunktion kann auch mehrfach im Molekül vertreten sein.

Als tertiäre Amine werden die in der DE-OS 25 07 461 aufgeführten Amine beispielhaft genannt.

Als tertiäre Amine werden in besonders günstiger Weise Tri-n-alkylamine wie Trimethylamin, Triethylamin, Tri-n-propylamin oder Tri-n-butylamin eingesetzt.

Das bevorzugte molare Verhältnis der Reaktionskomponenten (Alkanal bzw. Keton/ Formaldehyd/tertiäres Amin) beträgt 1/0,5-10/0,6-5, bevorzugt 1/2,5-6/0,8-2, insbesondere etwa 1/4,5/1,5.

Die Entwässerung des Reaktionsgemisches (Stufen (b), (c)) und die gleichzeitige Abtrennung der Leichtsieder, d.h. überschüssiges tertiäres Amin (beispielsweise Trialkylamin), überschüssiger Formaldehyd und das aus der Cannizzaro-Reaktion von Formaldehyd gebildete Methanol, kann in einer üblichen zur Destillation geeigneten Apparatur ein- oder mehrstufig durchgeführt werden. Für die diskontinuierliche Durchführung eignet sich beispielsweise ein Rotationsverdampfer, für die kontinuierliche Durchführung eignet sich beispielsweise ein Fallfilmverdampfer. Die Abtrennung kann unter vermindertem Druck oder bei Normaldruck oder in Gegenwart eines Inertgasstromes durchgeführt werden; die Abtrennung wird vorzugsweise bis zu einem Restgehalt an Wasser von 2-30 Gewichtsprozent bezogen auf die Reaktionslösung durchgeführt. Die Reaktionslösung enthält als hauptsächliche Komponenten den Polyalkohol, Polyalkoholhalbformale, bereits gebildete Polyalkoholformiate und Trialkylammoniumformiat.

Die Veresterung zu Polyalkoholformiaten (Stufe (c)) verläuft autokatalytisch durch weitere Abdestillation von (Reaktions)wasser, tertiärem Amin und dem aus den zuvor gebildeten Polyalkoholhalbformalen freiwerdenden Formaldehyd. Dabei wird aus Formiaten des tertiären Amins das Amin freigesetzt. Die Umsetzung kann diskontinuierlich, bevorzugt aber kontinuierlich, unter Normal- oder Unterdruck, zwischen 0,1 und 760 mbar durchgeführt werden. Die Reaktionstemperatur liegt zwischen 70°C und 250°C, bevorzugt zwischen 150°C und 210°C. Für die kontinuierliche Durchführung eignet sich eine Glockenbodenkolonne mit Umlaufverdampfer. Der Zulauf erfolgt im oberen Teil der Kolonne, die Verweilzeit im Abtriebsteil zusammen mit dem Umlaufverdampfer beträgt 0,5-5 Stunden, vorzugsweise 2-3 Stunden. Der freiwerdende Formaldehyd stört den Reaktionsverlauf nicht durch Bildung unlöslicher oligomerer Formaldehydablagerungen, da stets durch die Veresterungsreaktion Reaktionswasser gebildet wird, das den Formaldehyd gelöst hält.

Die Umesterung der Polyalkoholformiate (Stufe (e)) zu beispielsweise Alkylformiat und freiem Polyalkohol kann diskontinuierlich, z.B. in einem Rührkessel, oder kontinuierlich in Gegenwart von 0,5-20 Mol, vorzugsweise 1-15 Mol, insbesondere 1-4 Mol eines Alkohols pro Mol Polyalkoholformiat durchgeführt werden. Für die bevorzugte kontinuierliche Durchführung eignet sich eine Gegenstromkolonne, in der im mittleren Teil das Formiat, im unteren Teil ein Alkohol zudosiert wird. Im Abtriebsteil der Kolonne wird Alkylformiat von dem zugegebenen Alkohol getrennt, so daß am Kolonnenkopf weitgehend reines Alkylformiat kondensiert wird. Die Reaktionszeit beträgt 0,25-5 Stunden, bevorzugt 0,5-3 Stunden.

Alkohole, insbesondere Alkanole, mit 1-6 C-Atomen werden bevorzugt. Besonders geeignet sind Alkohole, bei denen die Siedepunktdifferenz zwischen Alkohol und entsprechendem Formiat möglichst hoch ist. Bevorzugt wird das Paar Methanol/Methylformiat und Ethanol/Ethylformiat verwendet.

Die Umesterung kann in Gegenwart der typischen homogenen oder inhomogenen Umesterungskatalysatoren durchgeführt werden. Als inhomogene Katalysatoren sind saure oder basische Alkali- oder Erdalkalioxide, Hydrotalcite oder Aluminiumoxid geeignet. Ebenso können saure oder basische Ionenaustauscher verwendet werden. Als homogene Katalysatoren eignen sich Alkali- oder Erdalkalialkoholate. Bei Verwendung von anorganischen Alkoholaten als Katalysator schließt sich eine Abtrennung des Katalysators durch Neutralisation oder Ionenaustausch nach der Umesterung an, was entweder Salzanfall oder zusätzlichen verfahrenstechnischen Aufwand bedingt. Bei Verwendung heterogener Katalysatoren ist die Standzeit dieser Katalysatoren zu berücksichtigen und eine mögliche Regenerierung notwendig.

Überraschend ist, daß die Umesterung in Gegenwart eines tertiären Amins, insbesondere Trialkylamin mit gutem Ergebnis durchgeführt werden kann. Dadurch kann der Katalysator nach der Reaktion einfach durch Destillation entfernt und wiederverwertet werden. Diese Möglichkeit besteht bei Katalysatoren auf Alkalimetall-Basis nicht. Als Trialkylamine eignen sich die in der Reaktionsstufe verwendeten Amine, wobei beispielsweise das gleiche Amin bei der Synthesereaktion wie auch bei der Umesterungsreaktion eingesetzt werden kann. Die Menge des eingesetzten Amins beträgt wenige Mol%, kann aber auch überstöchiometrisch sein. Sie beträgt vorzugsweise 1-50 Mol% bezogen auf 1 Mol Formiat als 100 Mol%. Das Amin wird gegebenenfalls durch Destillation quantitativ zurückgewonnen. Die durch tertiäres Amin katalysierte Umesterung verläuft äußerst schnell, so daß problemlos der notwendige quantitative Umsatz zu Alkylformiat und dem gewünschten Polyalkohol erzielt wird. Vorteilhaft gegenüber der Verwendung gebräuchlicher Umesterungskatalysatoren wie beispielsweise den Alkoholaten der Alkali- und Erdalkalimetalle, die durch Wasser hydrolysiert werden, ist, daß die Umsetzung in Gegenwart eines tertiären Amins wie beispielsweise Trialkylamin in wasserfreien aber auch verdünnten wäßrigen Lösungen durchgeführt werden kann.

Besonders vorteilhaft kann für die Umesterung (e) das Destillat aus der Abtrennung der Leichtsieder oder das bei der Bildung der Polyalkoholformiate (Stufen (b), (c), (d)) abdestillierte Gemisch aus Wasser, Trialkylamin und Formaldehyd, gegebenenfalls nach vorheriger Aufkonzentrierung und Abtrennung des Formaldehyds zur Umesterung verwendet werden. Die in diesen Gemischen enthaltene Menge an Methanol wird somit einerseits zur Bildung von Methylformiat genutzt, andererseits wird Methanol auf diese Weise aus den Gemischen durch chemische Reaktion entfernt. Da sich Alkohole in der Synthesestufe negativ auswirken können, muß vor einer Rückführung des Trialkylamin/Wasser/Formaldehydgemisches in die Synthesestufe Methanol abgetrennt werden. Dies ist mit Hilfe der beschriebenen Umesterungsreaktion möglich, aber nicht durch einfache Destillation ohne Verluste an Trialkylamin zu erreichen.

Aus der Umesterungsstufe wird eine alkoholische Lösung des gewünschten Polyalkohols erhalten (Stufe (f)). Durch Variation der verwendeten Menge an alkoholischem Lösungsmittel kann im Falle von kristallinen Polyalkoholen das Produkt direkt durch Kristallisation gewonnen werden. Der als Lösungsmittel verwendete Alkohol und tertiäres Amin als Katalysator können in die Umesterung (z.B. Stufen (a) und (e)) rückgeführt werden. Ebenso kann im Falle von gut destillierbaren Polyalkoholen zuerst der leichtsiedende Alkohol und tertiäres Amin abdestilliert und rückgeführt werden. Anschließend kann der Polyalkohol direkt gewonnen werden. Eine Neutralisation oder komplizierte Abtrennung des Umesterungskatalysators ist somit nicht erforderlich.

Vorzugsweise ist vorgesehen, daß in der Stufe (f) durch Trennung, vorzugsweise durch Destillation, der gewünschte Polyalkohol und ein Gemisch aus Alkohol und tertiärem Amin erhalten wird, das teilweise in Stufe (e) rückgeführt wird, und daß ein Teil dieses Gemisches aus Alkohol und tertiärem Amin in eine Stufe (g) überführt wird, in der abgetrenntes Material aus den Stufen (b) und (c) und dieser Teilstrom aus (f) in folgende drei Gemische getrennt werden, wobei die Trennung vorzugsweise destillativ durchgeführt wird:
1. ein Gemisch aus Alkohol/tertiärem Amin, das zumindest teilweise in Stufe (e) geführt wird,
2. ein Gemisch aus Wasser/tertiärem Amin/Formaldehyd, das zumindest teilweise in Stufe (a) geführt wird, und
3. eine mit Mittelsiedern angereicherte wäßrige Lösung.

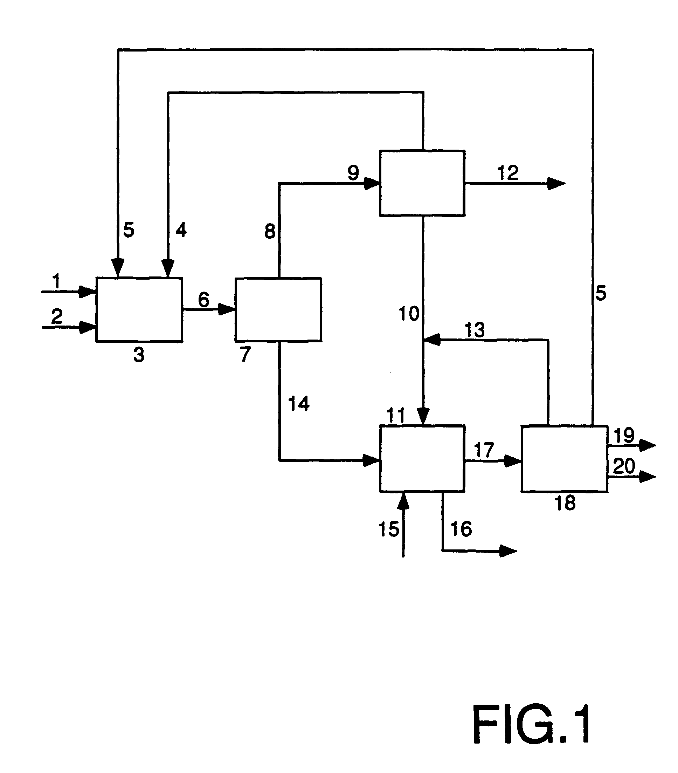

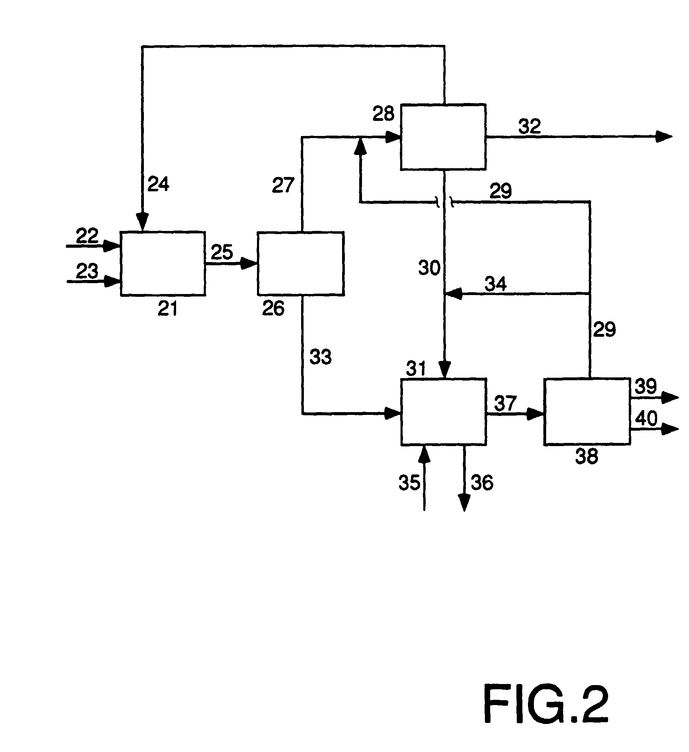

Beispielhaft werden im folgenden zwei vorteilhafte Ausgestaltungen des erfindungsgemäßen Verfahrens zur Herstellung von Polyalkoholen aus Alkanalen bzw. Ketonen und Formaldehyd unter Trialkylamin-Katalyse schematisch anhand der Verfahrensschemata gemäß Fig. 1 und Fig. 2 erläutert, wie sie z.B. bei der Herstellung von 1,1,1-Trimethylolpropan und Formaldehyd oder z.B. von Pentaerythrit aus Acetaldehyd und Formaldehyd in Gegenwart von z.B. Trimethylamin oder Triethylamin vorzugsweise angewandt werden. Aus Gründen der Übersichtlichkeit werden diese Ausgestaltungen im folgenden allein am Beispiel der Umsetzung von Alkanalen mit Formaldehyd geschildert.

In der Zeichnung zeigen Fig. 1 und Fig. 2 Verfahrensschemata des erfindungsgemäßen Verfahrens.

Im Verfahren gemäß Fig. 1 werden Formaldehyd und das betreffende Alkanal über die Leitungen 1 bzw. 2 in den Reaktor 3, der vorteilhaft eine Rührkessel-Kaskade ist, geleitet und dort mit über Leitung 4 rückgeführtem wäßrigem Formaldehyd, der gegebenenfalls noch geringe Mengen Trialkylamin enthalten kann, ergänzt sowie über Leitung 5 mit einer rückgeführten wäßrigen Lösung des Trialkylaminkatalysators versetzt. Bei der Umsetzung im Reaktor 3 bildet sich der in α-Position zur Aldehydgruppe je nach verwendetem Ausgangsmaterial bis- oder tris-hydroxymethyl-substituierte Aldehyd, der noch im Reaktor 3 mit weiterem Formaldehyd in einer gekreuzten Cannizzaro-Reaktion zur entsprechenden Tris- oder Tetrahydroxymethyl-Verbindung - in dieser Anmeldung auch als Trismethylolverbindung bezeichnet (im folgenden wird aus Gründen der Übersichtlichkeit nur von der Trimethylolverbindung gesprochen) - abreagiert, wobei der Formaldehyd in Ameisensäure umgewandelt wird und mit dem Trialkylamin-Katalysator Trialkylammoniumformiatsalze bildet. Der Austrag aus Reaktor 3 wird über Leitung 6 dem mittleren Teil einer Destillationskolonne 7 zugeführt, die vorteilhaft mit einem Umlaufverdampfer ausgestattet sein kann, worin leichtsiedende Bestandteile des Austrags aus Reaktor 3, wie Wasser, Formaldehyd, das Trialkylamin und bei der Umsetzung in Reaktor 3 durch Cannizzaro-Reaktion entstandenes Methanol, über Kopf von den höhersiedenden Bestandteilen des Austrags aus Reaktor 3, wie dem 1,1,1-Trimethylolalkan und Trimethylolalkanameisensäureestern - letztere werden in der Destillationskolonne durch Reaktion des Trialkylammoniumformiat-Salzes mit dem 1,1,1-Trimethylolalkan gebildet, wobei der Trialkylamin-Katalysator wieder freigesetzt wird - abdestilliert werden. Vorteilhaft kann der Austrag aus Reaktor 3 vor Einleitung in die Kolonne 7 eingeengt werden, beispielsweise in einem nicht in Fig. 1 eingezeichneten Dünnschicht- oder Fallfilmverdampfer.

Die in Kolonne 7 abgetrennten leichtsiedenden Bestandteile des Austrags aus Reaktor 3 werden über Leitung 8 einer weiteren Kolonne 9 zugeführt, wo sie destillativ in drei Fraktionen aufgetrennt werden, nämlich a) eine relativ niedrigsiedende Fraktion, die im wesentlichen aus dem Trialkylamin, Methanol und Wasser besteht und über Leitung 10, gegebenenfalls nach Ergänzung mit einem weiteres Trialkylamin, Wasser und Methanol enthaltenden Rückführstrom aus Leitung 13, dem unteren Teil einer weiteren Kolonne 11 zugeführt wird, b) eine mittelsiedende, im wesentlichen aus Wasser und nicht umgesetztem Formaldehyd bestehende Fraktion, die noch geringe Mengen des Trialkylamins enthalten kann und über Leitung 4 in den Reaktor 3 zurückgeleitet wird, und c) eine mit Nebenprodukten, wie Ethylacrolein, angereicherte hochsiedende, wäßrige Fraktion, welche über Leitung 12 aus dem Verfahren ausgeschleust wird.

Der hochsiedende, das 1,1,1-Trimethylolalkan und den Ameisensäureester des 1,1,1-Trimethylolalkans enthaltende Sumpfaustrag aus Kolonne 7 wird über Leitung 14 in den mittleren Teil der Kolonne 11 gepumpt. Da das 1,1,1-Trimethylolalkan und dessen Ameisensäureester höher sieden als das im unteren Teil der Kolonne 11 über Leitung 10 bzw. Leitung 13 zugeführte Gemisch aus Trialkylamin, Methanol und Wasser - das Gemisch aus Leitung 13 kann der Kolonne 11 über einen separaten, in Fig. 1 nicht eingezeichneten Einlaß oder nach vorheriger Vereinigung mit dem Strom in Leitung 10 zugeführt werden - und das zusätzlich über Leitung 15 in den unteren Teil der Kolonne 11 eingebrachte Methanol, kommen die relativ hoch siedenden Verbindungen 1,1,1-Trimethylolalkan und dessen Ameisensäureester in Kolonne 11 mit den bei niedrigerer Temperatur siedenden Komponenten des in den unteren Teil der Kolonne eingeleiteten Trialkylamin/Methanol/Wasser-Gemisches in intensiven Kontakt, wobei der Ameisensäureester des 1,1,1-Trimethylolalkans vollständig mit dem bezüglich des Ameisensäureesters in überschüssiger Menge in der Kolonne 11 vorhandenen Methanol zu 1,1,1-Trimethylolalkan und dem sehr niedrig siedenden Methylformiat umgeestert wird. Das Methylformiat wird am Kopf der Kolonne 11 über Leitung 16 aus dem Verfahren ausgeschleust und der weiteren Verwendung, beispielsweise der Herstellung von Ameisensäure oder Formamid, zugeführt.

Das von niedrigsiedendem Methylformiat befreite Umesterungsgemisch der Kolonne 11, das im wesentlichen aus dem Trialkylamin, überschüssigem Methanol, Wasser und dem 1,1,1-Trimethylolalkan besteht, wird im Sumpf der Kolonne 11 abgezogen und über Leitung 17 in den mittleren Teil der Kolonne 18 geleitet, in der dieses Gemisch in vier Fraktionen aufgetrennt wird, nämlich a) ein relativ niedrig siedendes, im wesentlichen aus Trialkylamin, Wasser und Methanol bestehendes Gemisch, das am Kopf der Kolonne 18 abgezogen und über Leitung 13 wieder in die Kolonne 11 zurückgeführt wird, b) ein im wesentlichen aus Trialkylamin und Wasser bestehendes Gemisch, das im oberen Teil der Kolonne abgeführt und über Leitung 5 in den Reaktor 3 zurückgeleitet wird, c) eine im wesentlichen aus dem 1,1,1-Trimethylolalkan bestehende Fraktion, die im unteren Teil der Kolonne 18 abgetrennt und über Leitung 19 z.B. dem Tanklager zugeführt wird, und d) ein aus hochsiedenden Nebenprodukten bestehendes Gemisch, das am Sumpf der Kolonne 18 abgezogen und zur Abtrennung von den Hochsiedern einer weiteren, in Fig. 1 nicht eingezeichneten Destillationskolonne zugeführt werden.

Somit ist der Kreislauf der Verfahrensausgestaltung des erfindungsgemäßen Verfahrens gemäß Fig. 1 geschlossen. Zum Ausgleich von Verlusten an Trialkylamin im Zuge des Verfahrens wird dieses durch Zuführung frischen Trialkylamins, vorzugsweise durch dessen Zugabe in den Reaktor 3 über einen nicht in Fig. 1 eingezeichneten Zulauf, ersetzt.

In einer anderen vorteilhaften Ausgestaltung des erfindungsgemäßen Verfahrens zur Herstellung von 1,1,1-Trimethylolalkanen aus Alkylalkanalen und Formaldehyd, wie sie schematisch in Fig. 2 dargestellt ist, wird dem Reaktor 21 über Leitung 22 wäßrige Formaldehydlösung, über Leitung 23 das Alkanal und über Leitung 24 ein rückgeführtes Trialkylamin/Formaldehyd/Wasser-Gemisch zugeführt. Bei Bedarf kann über einen nicht in Fig. 2 eingezeichneten Zulauf im Zuge des Verfahrens verbrauchtes Trialkylamin durch frisches Trialkylamin ersetzt werden.

Bei der Umsetzung im Reaktor 21, der im wesentlichen gleich dem Reaktor 3 von Fig. 1 ausgestaltet ist, bildet sich ein Produktgemisch von im wesentlichen gleicher Zusammensetzung wie in Reaktor 3, das über Leitung 25, gegebenenfalls nach vorheriger Einengung z.B. in einem nicht eingezeichneten Fallfilm- oder Dünnschichtverdampfer, dem mittleren Teil der Kolonne 26 zugeleitet, die praktisch der Kolonne 7 in Fig. 1 entspricht, wo der Austrag aus Reaktor 21, wie bei der Verfahrensbeschreibung zu Fig. 1 beschrieben, verestert und in eine leichtersiedende, im wesentlichen aus Trialkylamin, Methanol, Formaldehyd und Wasser bestehende Fraktion, die am Kopf der Kolonne 26 abgezogen und über Leitung 27 einer weiteren Kolonne 28 zugeführt wird und in eine höhersiedende, im wesentlichen aus dem 1,1,1-Trimethylolalkan und dessen Ameisensäureester bestehenden Sumpf-fraktion, aufgetrennt wird.

Das in Leitung 27 der Kolonne 28 zugeführte Gemisch wird zweckmäßigerweise vor seiner Einleitung in Kolonne 28 mit einem über Leitung 29 rückgeführten, im wesentlichen aus Trialkylamin und Methanol zusammengesetzten Gemisch, das alternativ aber auch über einen separaten, in Fig. 2 nicht eingezeichneten Zulauf in die Kolonne 28 gegeben werden kann, vereinigt.

Im Unterschied zur Destillationsweise in Kolonne 9 in der Verfahrensausgestaltung gemäß Fig. 1 wird das der Kolonne 28 aufgegebene Gemisch dergestalt in drei Fraktionen aufgetrennt, daß a) am Kopf der Kolonne eine relativ niedrig siedende Fraktion, die im wesentlichen aus Trialkylamin und Methanol zusammengesetzt ist und praktisch kein Wasser mehr enthält, abgezogen und über Leitung 30 dem unteren Teil einer weiteren Kolonne 31 zugeführt wird, b) im oberen Teil der Kolonne 28 eine Fraktion, die im wesentlichen aus einem Gemisch der Komponenten Trialkylamin, Formaldehyd und Wasser besteht, geschnitten wird, die über Leitung 24 in den Reaktor 21 zurückgeleitet wird, und c) eine mit Nebenprodukten, wie Ethylacrolein, angereicherte höhersiedende, wäßrige Fraktion über Leitung 32 aus dem Verfahren ausgeschleust wird.

Der hochsiedende, im wesentlichen aus dem 1,1,1-Trimethylolalkan und dessen Ameisensäureester bestehende Sumpfaustrag aus Kolonne 26 wird über Leitung 33 in den mittleren Teil der Kolonne 31 gepumpt. Da das 1,1,1-Trimethylolalkan und dessen Ameisensäureester höher sieden als das im unteren Teil der Kolonne 31 über Leitung 30 bzw. Leitung 34 zugeführte Gemisch aus Trialkylamin und Methanol - das Gemisch aus Leitung 34 kann der Kolonne 31 über einen separaten, in Fig. 2 nicht eingezeichneten Zulauf oder zweckmäßigerweise nach vorheriger Vereinigung mit dem Strom in Leitung 30 zugeführt werden - und das zusätzlich über Leitung 35 in den unteren Teil der Kolonne 31 eingebrachte Methanol, kommen die relativ hoch siedenden Verbindungen 1,1,1-Trimethylolalkan und dessen Ameisensäureester in Kolonne 31 mit den Komponenten des bei niedriger Temperatur siedenden, in den unteren Teil der Kolonne 31 eingeleiteten Trialkylamin/Methanol-Gemisches in innigen Kontakt, wobei der Ameisensäureester des 1,1,1-Trimethylolalkans praktisch quantitativ mit dem bezüglich des Ameisensäureesters in überschüssiger Menge in der Kolonne 31 vorhandenen Methanol zu 1,1,1-Trimethylolalkan und dem sehr niedrig siedenden Methylformiat umgeestert wird. Das Methylformiat wird am Kopf der Kolonne 31 über Leitung 36 aus dem Verfahren ausgeschleust.

Das vom niedrigsiedenden Methylformiat befreite Umesterungsgemisch der Kolonne 31, das im wesentlichen aus dem Trialkylamin, überschüssigem Methanol und dem 1,1,1-Trimethylolalkan zusammengesetzt ist, wird im Sumpf der Kolonne 31 abgezogen und über Leitung 37 in den mittleren Teil der Kolonne 38 geleitet, in der dieses Gemisch in drei Fraktionen aufgetrennt wird, nämlich a) ein relativ niedrig siedendes, im wesentlichen aus Methanol und Trialkylamin bestehendes Gemisch, das am Kopf der Kolonne 38 abgezogen und das nach Abtrennung eines Teilstroms durch Leitung 34, über die Leitungen 29 und 27 in die Kolonne 28 zurückgeführt wird, b) eine im wesentlichen aus dem 1,1,1-Trimethylolalkan bestehende Fraktion, die im unteren Teil der Kolonne 38 abgetrennt und über Leitung 39 z.B. dem Tanklager zugeführt wird, und c) ein aus hochsiedenden Nebenprodukten bestehendes Gemisch, das am Sumpf der Kolonne 38 über Leitung 40 aus dem Verfahren ausgeschleust wird.

Der aus Leitung 29 in Leitung 34 abgezweigte Methanol- und Trialkylaminhaltige Teilstrom wird zweckmäßigerweise mit dem Methanol-Trialkylamin-Strom in Leitung 30 vereinigt und mit diesem, wie beschrieben, der Kolonne 31 zugeführt. Die Menge des über Leitungen 34 und 30 in die Umesterungskolonne zurückgeführten Teilstroms wird selbstverständlich so eingeregelt, daß sich in der Anlage ein stationäres Gleichgewicht, entsprechend den angewandten, im vorausgehenden Teil der Beschreibung spezifizierten Reaktions- und Destillationsbedingungen, einstellt.

### BEISPIEL 1

Man leitet in eine kontinuierlich betriebene Reaktorkaskade aus drei hintereinandergeschalteten Kolben mit Rührer (Reaktionsvolumen jeweils 300 ml) stündlich 32,1 ml Triethylamin (1,5 Mol), 13,84 ml n-Butyraldehyd (1 Mol) und 134,2 ml Formaldehyd (15%ig, 4,5 Mol). Die Rührkolben werden folgendermaßen temperiert: Kolben 1 = 42°C; Kolben 2 = 60°C, Kolben 3 = 78°C. Die GC-analytisch bestimmte Rohausbeute beträgt 89% TMP (Trimethylolpropan), der Ameisensäuregehalt liegt bei 5,2%.

Beispiele 2-9 in Tabelle 1 wurden bei gleicher Temperaturführung mit verschiedenen Konzentrationen der Einsatzstoffe analog Beispiel 1 durchgeführt.

### BEISPIEL 10

Der Rohaustrag aus Beispiel 1 wird zur Abtrennung der Leichtsieder und Wasser in einen Fallfilmverdampfer, der auf 150°C temperiert ist, bei Raumdruck gepumpt. Der Zulauf beträgt 120 ml/h, der Sumpfablauf beträgt 43 g/h, die Destillatmenge beträgt 80 g/h. Das Destillat ist zweiphasig; Destillatzusammensetzung: Wasser 87%, Triethylamin 8,5%, Formaldehyd 3,5%, Methanol 1%.

### BEISPIELE 11 BIS 14

Es wird wie in Beispiel 10 vorgegangen. Die Ergebnisse sind in Tabelle 2 zusammengestellt.

### BEISPIEL 15

Ein Gemisch aus Wasser (21%), Ameisensäure (12%), Triethylamin (27%), TMP (37%) und Formaldehyd (1%) wird auf den 8ten Boden einer 10-bödigen Glockenbodenkolonne (V = 300 ml) gepumpt (Zulauf 214 ml/h), die bei einem Kopfdruck von 63 mbar und einer Sumpftemperatur von 194°C betrieben wird. Der Sumpfablauf wird mit einem Umlaufverdampfer (Reaktionsvolumen = 150 ml) beheizt, aus dem kontinuierlich 100 g/h TMP-Formiate abgepumpt werden. Das Kondensat ist zweiphasig.

### BEISPIELE 16 BIS 18

Es wird im Prinzip wie in Beispiel 15 gearbeitet. Die Abwandlungen und Ergebnisse sind in Tabelle 3 zusammengestellt.

### BEISPIEL 19

Der Sumpfablauf aus Beispiel 10 (Wasser 12,5%, Ameisensäure 13,5%, Triethylamin 35%, Trimethylolpropan und Trimethylolpropanhalbformale 30%, Dimer-TMP 3%, Bis-TMP-Formaldehyd-acetal 4%, Rest (Hoch- und Mittelsieder) 2%) wird auf den 8ten Boden einer 10-bödigen Glockenbodenkolonne (V = 300 ml) gepumpt (Zulauf 150 ml/h), die bei einem Kopfdruck von 32 mbar und einer Sumpftemperatur von 194°C betrieben wird. Der Sumpfablauf wird mit einem Umlaufverdampfer (Reaktionsvolumen = 150 ml) beheizt, aus dem kontinuierlich 62 g/h TMP-Formiate abgepumpt werden. Das Kondensat (87g) ist zweiphasig;
Destillatzusammensetzung: Wasser (55%), Formaldehyd (5,2%), Triethylamin (36,5%), Mittelsieder (3,3%).
Sumpfzusammensetzung: TMP (29%), TMP-monoformiat (30%), TMP-diformiat (25%), TMP-triformiat (5%), Hochsieder (11%), u.a. Di-TMP und Di-TMP-formiate, Formaldehyd-bis(TMP)acetal und Formaldehyd-bis(TMP)acetal-formiate.

### BEISPIEL 20

Ein Gemisch aus TMP, TMP-monoformiat, TMP-diformiat und TMP-triformiat (37/42/19/2) wird zusammen mit 1 Gew % Natriummethanolat auf den 6ten Boden einer 10bödigen Glockenbodenkolonne (V = 360 ml) gepumpt (133 ml/h). Über den Sumpfkolben (Reaktionsvolumen = 360 ml) wird kontinuierlich 111 ml/h Methanol zudosiert. Über eine Pumpe werden 180 ml/h Reaktionsgemisch aus dem Sumpf entfernt, am Kolonnenkopf werden 53 ml/h Destillat gewonnen. Die Zusammensetzung der Austräge sind in Tabelle 4 zusammengestellt. Beispiel 21 und 22 wurden analog durchgeführt.

### BEISPIELE 23 UND 25

Es wird im Prinzip wie in Beispiel 20 gearbeitet, mit der Ausnahme jedoch, daß anstelle von Natriummethanolat Triethylamin zugegeben wird Tabelle 4 zeigt die Arbeitsbedingungen und Ergebnisse.

### BEISPIEL 26

176 g des Sumpfablaufs aus Beispiel 19 werden mit 300 g Methanol und 9 g Triethylamin in einem 1 1 Rührkolben 30 Minuten bei 40°C gerührt. Anschließend wird über eine kurze Kolonne zuerst Methylformiat, dann Methanol und Triethylamin abdestilliert. Danach werden in derselben Apparatur bei einem Vakuum von 2 mbar folgende Fraktionen erhalten:

| | | | |
|---|---|---|---|
| I | Sdp. 120-136°C | 4g | 80%TMP (GC) |
| II | Sdp. 140-160°C | 124g | 99,3%TMP (GC) |
| III | Sdp. 218-225°C | 20g | 14% TMP, 86% Di-TMP und TMP FA TMP |
| IV | Rückstand | 2g | |

Fraktion II weist eine OH-Zahl von 1232 auf. Die Ausbeute an TMP bezüglich n-Butyraldehyd beträgt über alle Reaktionsstufen (Beispiel 1, Beispiel 10, Beispiel 19) 87,8%.

### BEISPIEL 27

2167 g Rohaustrag aus Beispiel 1 werden wie im Beispiel 10 über einem Fallfilmverdampfer entwässert und zusammen mit überschüssigem Formaldehyd und überschüssigem Triethylamin abgetrennt. Destillat: 1390 g, Sumpf: 774 g.

In einem 11 Kolben werden obige 774 g Sumpfablauf erhitzt und Leichtsieder über einer Vigreuxkolonne abdestilliert. Die Temperatur wird von 140°C auf 180°C erhöht. Zuletzt wird ein Vakuum von 300 mbar angelegt. Destillat: 411 g, Sumpf: 351 g.

Das erhaltene Formiatgemisch (351g) wird in der selben Apparatur mit 238g Methanol und 1,8 g Natriummethanolat versetzt und bei Normaldruck werden 295 g eines Gemisches aus Methylformiat und Methanol abdestilliert. Eine anschließend durchgeführte Destillation ergibt folgende Fraktionen:

| | | | |
|---|---|---|---|
| I | Sdp.₄ 110-125°C | 2,4g | 80% TMP (GC) |
| II | Sdp.₄ 148-160°C | 227,3g | 99,3% TMP (GC) |
| III | Rückstand | 65,4g | |

Die TMP-Ausbeute über alle Aufarbeitungsstufen (bzgl. n-Butyraldehyd) beträgt 87,9% (91% mit TMP im Rückstand).

### BEISPIEL 28

Man leitet in eine kontinuierlich betriebene Reaktionskaskade aus drei hintereinandergeschalteten Kolben mit Rührer (Reaktionsvolumen jeweils 300 ml) stündlich 32,4 ml Triethylamin (1,5 Mol), 11,1 ml n-Propanal (1 Mol) und 147 ml Formaldehyd (15%ig, 4,5 Mol). Die Rührkolben werden folgendermaßen temperiert: Kolben 1 = 42°C, Kolben 2 = 60°C, Kolben 3 = 78°C.

3800 g des so erhaltenen Austrags werden am Rotationsverdampfer bis auf 1010 g eingeengt. Anschließend wird der Rückstand allmählich auf 180°C bei Normaldruck erhitzt und zuletzt bei dieser Temperatur ein Vakuum von 300 mbar angelegt. Wasser, Formaldehyd und Triethylamin werden über eine Vigreuxkolonne abdestilliert. Der Rückstand wird mit 400 ml Methanol versetzt, und es werden 40 g Triethylamin hinzugegeben. Anschließend werden Methylformiat, Methanol und Triethylamin abdestilliert. Die verbleibende methanolische Lösung wird bis zu einem 50%igen Gewichtsanteil an Trimethylolethan aufkonzentriert. Durch Zugabe von Methylisobutylketon fällt Trimethylolethan aus, aus der Mutterlauge wird durch weiteres Aufkonzentrieren noch zweimal Trimethylolbutan ausgefällt; insgesamt werden 342 g Trimethylolethan erhalten. Die Ausbeute (bzgl. n-Propanal) beträgt 75%.

### BEISPIEL 29

Man leitet in eine kontinuierlich betriebene Reaktionskaskade aus drei hintereinandergeschalteten Kolben mit Rührer (Reaktionsvolumen jeweils 300 ml) stündlich 31,6 ml Triethylamin (1,5 Mol), 15,5 ml n-Pentanal (1 Mol) und 143 ml Formaldehyd (15%ig, 4,5 Mol). Die Rührkolben werden folgendermaßen temperiert: Kolben 1 = 42°C, Kolben 2 = 70°C, Kolben 3 = 78°C.

3845 g des so erhaltenen Austrags werden am Rotationsverdampfer eingeengt. Anschließend wird der Rückstand wie im Beispiel 28 erhitzt, wobei Wasser, Formaldehyd und Triethylamin über eine Vigreuxkolonne abdestilliert werden. Der Rückstand wird mit 400 ml Methanol versetzt und 40 g Triethylamin hinzugegeben. Anschließend werden Methylformiat, Methanol und Triethylamin abdestilliert. Die verbleibende Lösung wird durch Destillation in 3 Fraktionen aufgetrennt.

| | | | |
|---|---|---|---|
| I | Vorlauf | 21 g | 30% Trimethylolbutan (GC) |
| II | Sdp.₁ 155-160°C | 283 g | 98% Trimethylolbutan (GC) |
| III | Sdp.₂ 195-198°C | 125 g | 7,4% Trimethylolbutan; |
| | | | 74% Formaldehyd-bis(TMB)-acetal |

Die Ausbeute an Trimethylolbutan (TMB) bzgl. n-Pentanal beträgt 58%, die Ausbeute an Formaldehyd-bis(TMB)-acetal beträgt 19%.

### BEISPIEL 30

Man leitet in eine kontinuierlich betriebene Reaktionskaskade aus drei hintereinandergeschalteten Kolben mit Rührer (Reaktionsvolumen jeweils 300 ml) stündlich 27,6 ml Triethylamin (1,5 Mol), 45,2 ml wäßrigen Acetaldehyd (13,8%ig, 1 Mol) und 115,2 ml Formaldehyd (20%ig, 5,5 Mol). Die Rührkolben werden folgendermaßen temperiert: Kolben 1 = 27°C, Kolben 2 = 30°C, Kolben 3 = 40°C.

4376 g des so erhaltenen Austrags werden am Rotationsverdampfer eingeengt. Anschließend wird der Rückstand allmählich auf 180°C bei Normaldruck erhitzt und zuletzt bei dieser Temperatur ein Vakuum von 300 mbar angelegt. Wasser, Formaldehyd und Triethylamin und Mittelsieder werden über eine Vigreuxkolonne abdestilliert. Der Rückstand wird auf 60°C abgekühlt und mit 400 ml Methanol versetzt. Nach Zugabe von 40 g Triethylamin werden Methylformiat, Methanol und Triethylamin abdestilliert. Aus der verbleibenden methanolischen Lösung fällt bereits Pentaerythrit aus, das über einen Büchnertrichter abgesaugt wird. Die Mutterlauge wird von Methanol befreit und mit Aceton versetzt. Dabei wird weiteres Pentaerythrit erhalten. Insgesamt werden 283 g erhalten. Die Ausbeute (bzgl. Acetaldehyd) beträgt 61%.

### BEISPIEL 31

In einem Dreihalskolben werden 31 ml Cyclohexanon (0,3 Mol) und 165 ml 30%iger Formaldehyd (1,5 Mol) vorgelegt. Dazu tropft man 62 ml Triethylamin (0,45 Mol), worauf Erwärmung auf 60°C eintritt. Es wird noch weitere 6 h bei dieser Temperatur gerührt. Der Umsatz bezüglich Cyclohexanon beträgt 90%. Das Reaktionsgemisch wird am Rotationsverdampfer eingeengt und anschließend bei 180°C erhitzt, um nicht umgesetztes Cyclohexanon, Wasser, Triethylamin und Formaldehyd abzudestillieren. Zuletzt wird bei 180°C ein Vakuum von 300 mbar angelegt.

Der Rückstand von 70 g wird in 100 ml Methanol aufgenommen und mit 7 g Triethylamin versetzt. Anschließend wird gemeinsam ein Gemisch aus Methylformiat, Methanol und Triethylamin abdestilliert. Es verbleiben 150 ml methanolische Lösung, aus der bereits 2,2,6,6-Tetrakis(hydroxymethyl)cyclohexanol beim Abkühlen ausfällt. Die verbleibende Lösung wird vom restlichen Methanol am Rotationsverdampfer befreit und aus dem Rückstand wird mit Aceton weiteres 2,2,6,6-Tetrakis(hydroxymethyl)-cyclohexanol ausgefällt. Insgesamt werden 30 g erhalten.
Ausbeute: 61% bezogen auf umgesetztes Cyclohexanon.

In den folgenden Tabellen sind Einzelheiten und Ergebnisse aus den vorherigen Beispielen zusammengestellt.

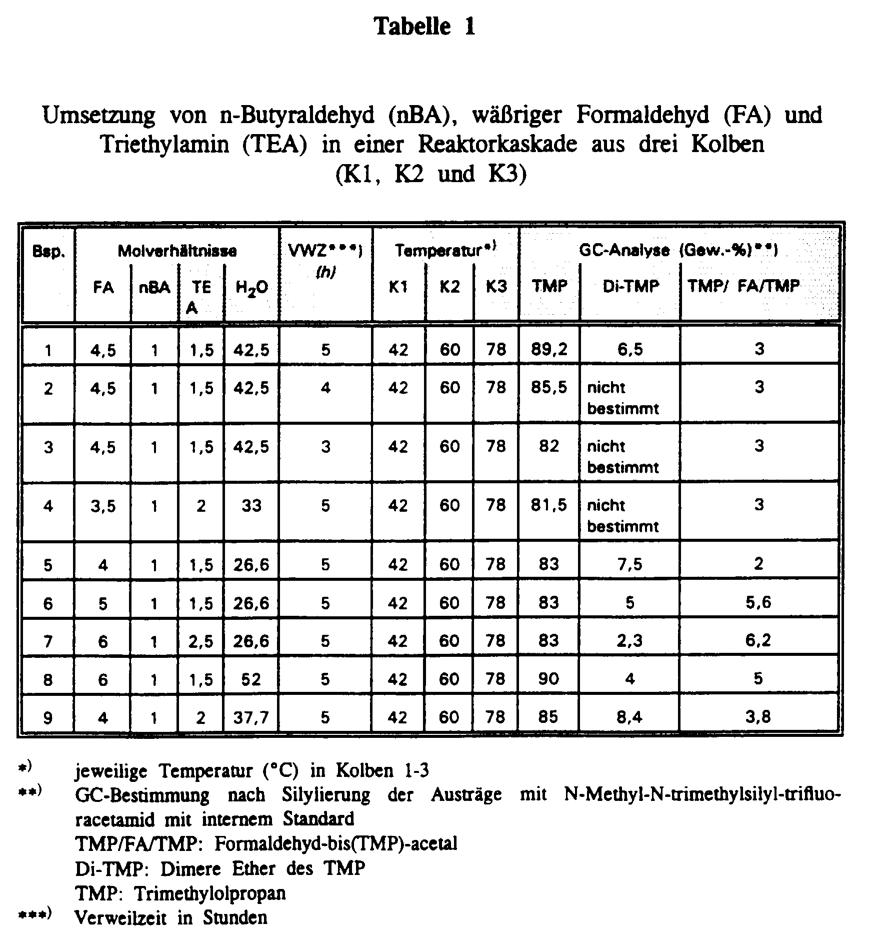

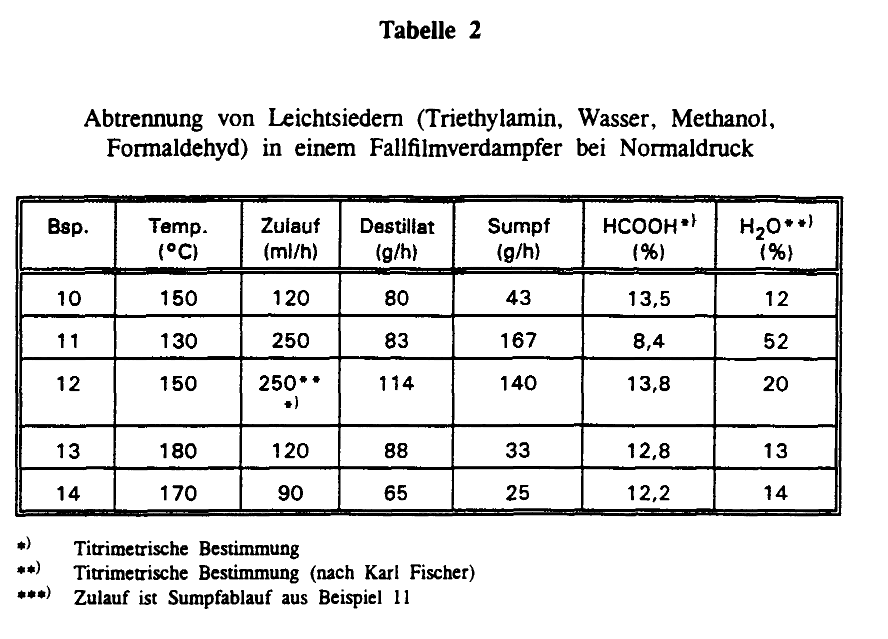

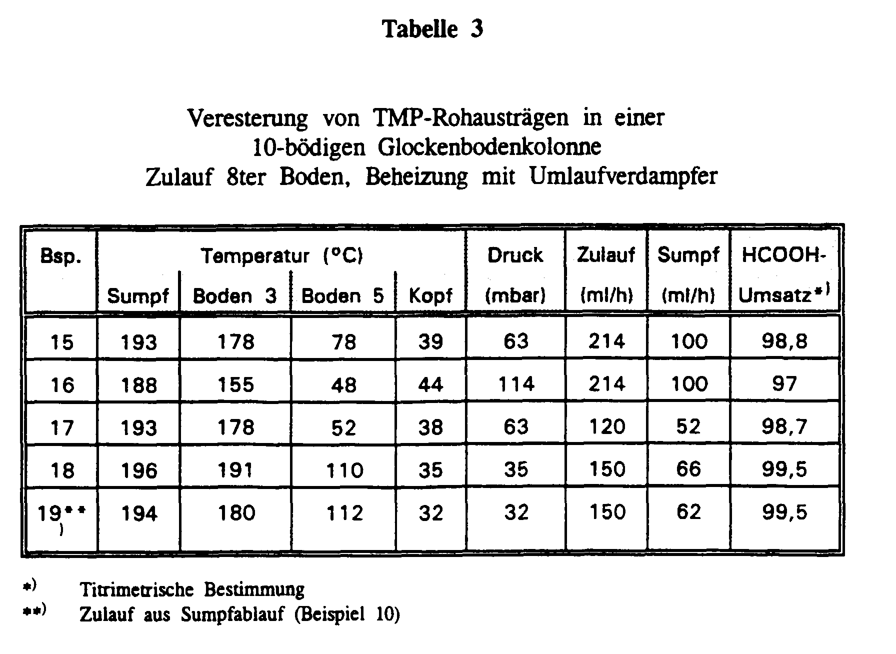

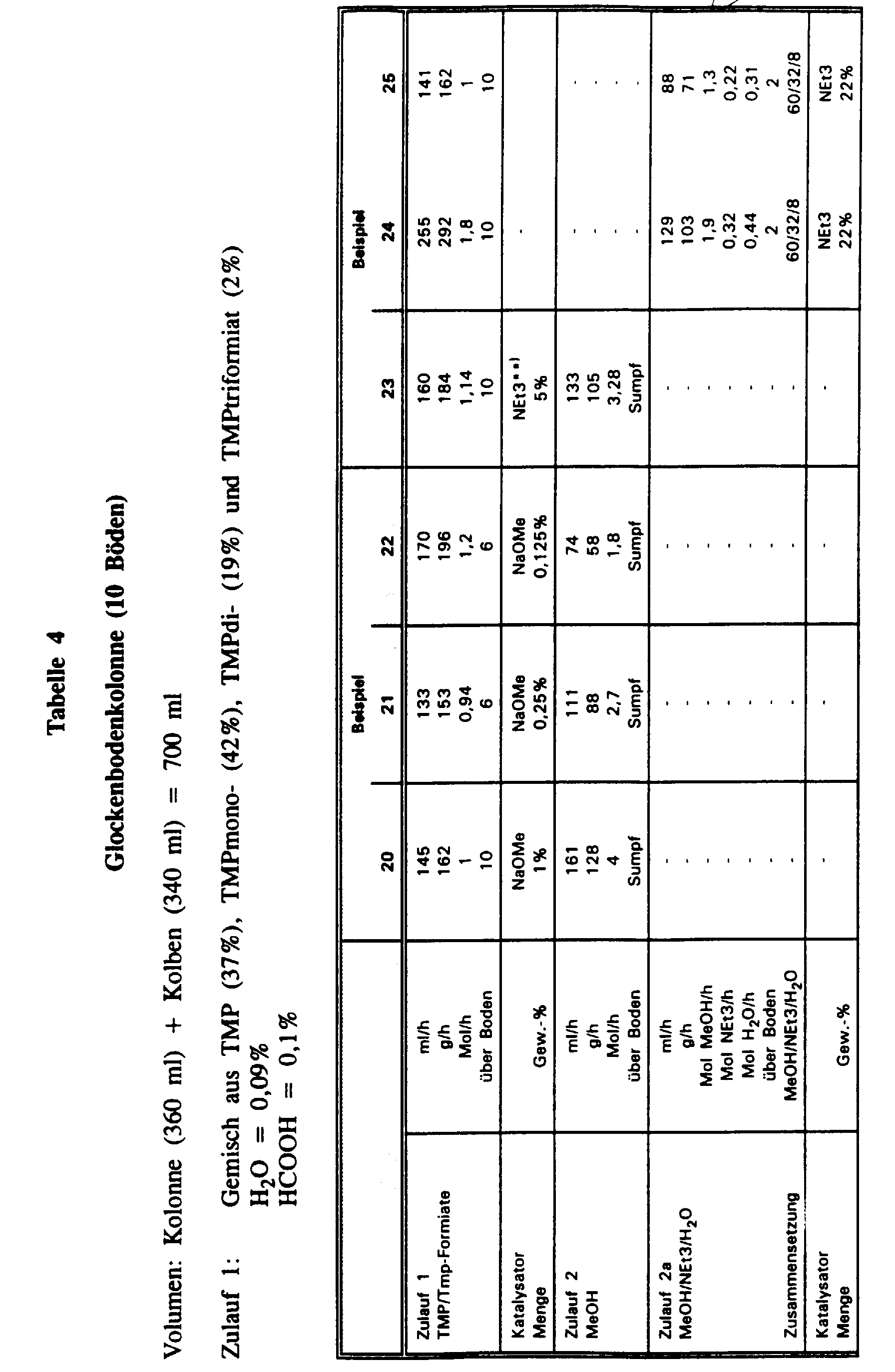

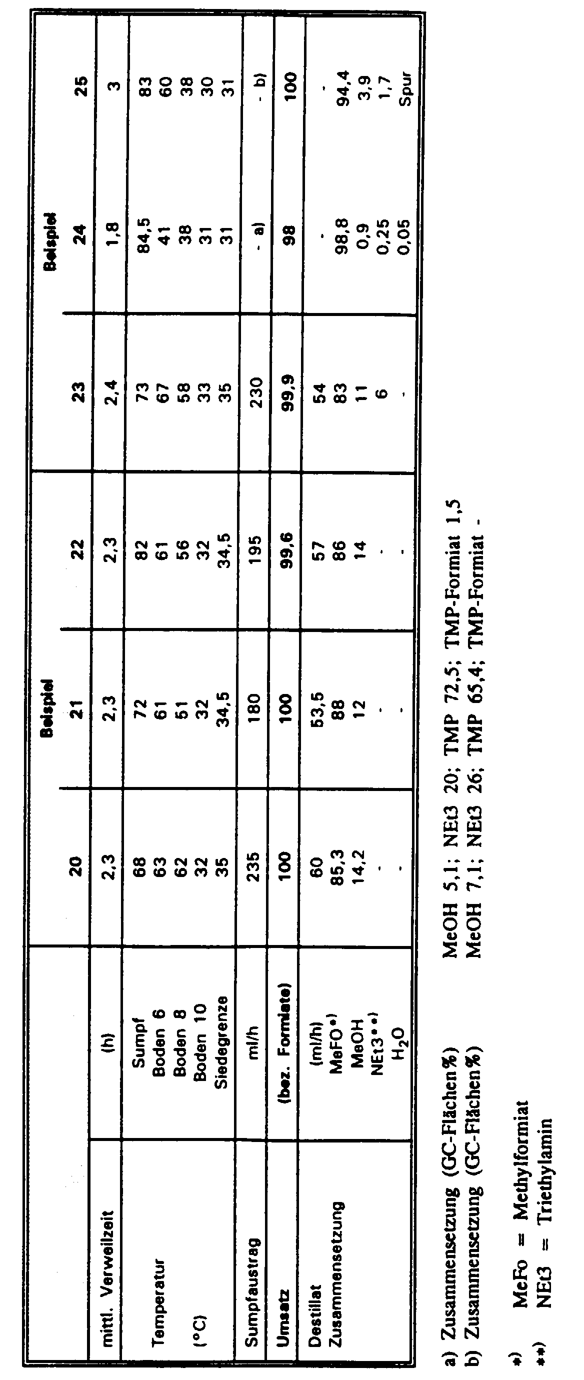

## Patentansprüche

1. Verfahren zur Herstellung eines Formiate enthaltenden Polyalkoholprodukts durch Umsetzung eines Alkanals oder Ketons mit Formaldehyd in wäßriger Lösung in Gegenwart von tertiärem Amin, dadurch gekennzeichnet, daß die Umsetzung temperaturkaskadiert mit steigender Temperatur, und während einer Dauer von 0,5-24 Stunden durchgeführt wird und bezogen auf 1 Mol Alkanal oder Keton 0,6 bis 5 Mol tertiäres Amin eingesetzt werden und das Verfahren ohne Hydrierung des Produktgemisches durchgeführt wird.

2. Verfahren zur Herstellung von Polyalkoholen, welches die Stufen umfaßt:
(a) Umsetzung eines Alkanals oder Ketons mit Formaldehyd in wäßriger Lösung in Gegenwart eines tertiären Amins zu einem Formiate enthaltenden Polyalkoholproduktgemisch gemäß dem Verfahren nach Anspruch 1,
(b) Abtrennung von Wasser, überschüssigem tertiärem Amin, überschüssigem Formaldehyd,
(c) Erhitzen des verbliebenen Gemisches von (b) unter Abtrennung von weiterem Formaldehyd und tertiärem Amin und Bildung von Polyalkoholformiaten,
(d) Überführung von abgetrenntem tertiärem Amin aus der Stufe (b) und/oder der Stufe (c) in die Synthesestufe (a) und/oder in die folgende Umesterungsstufe (e),
(e) Umesterung von erhaltenen Polyalkoholformiaten aus der Stufe (c) mit einem Alkohol der Formel ROH in Gegenwart eines Umesterungskatalysators zu Polyalkoholen und Formiaten der Formel 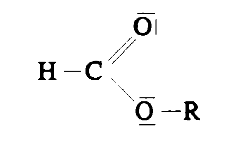 wobei R einen Kohlenwasserstoffrest, bedeutet,
(f) Gewinnung von Polyalkoholen.

3. Verfahren nach Anspruch 2, dadurch gekennzeichnet, daß der Umesterungskatalysator ein tertiäres Amin ist.

4. Verfahren nach Anspruch 2 oder 3, dadurch gekennzeichnet, daß die Umesterung in Gegenwart eines Trialkylamins als Umesterungskatalysator durchgeführt wird, wobei die Menge des eingesetzten Trialkylamins im Bereich von 0,01-75 Mol%, bezogen auf 1 Mol Formiat des Polyalkoholformiats als 100 Mol%, liegt.

5. Verfahren nach einem der Ansprüche 2 bis 4, dadurch gekennzeichnet, daß in einer Stufe (g) das abgetrennte Material aus den Stufen (c) und (b) in die folgenden Gemische getrennt wird:
1. ein Gemisch aus Alkohol/tertiärem Amin/Wasser, das zumindest teilweise in Stufe (e) geführt wird,
2. ein Gemisch aus Wasser und Formaldehyd, das zumindest teilweise in Stufe (a) geführt wird, und
3. eine mit Mittelsiedern angereicherte wäßrige Lösung.

6. Verfahren nach einem der Ansprüche 2 bis 5, dadurch gekennzeichnet, daß das Verfahren ohne Hydrierung des Produktgemisches der Stufe (a) durchgeführt wird.

7. Verfahren nach einem der Ansprüche 1 bis 6, dadurch gekennzeichnet, daß das Verhältnis der Reaktionskomponenten bezogen auf 1 Mol Alkanal bzw. Keton im Bereich von 0,5-10 Mol Formaldehyd liegt.

8. Verfahren nach einem der Ansprüche 1 bis 7, dadurch gekennzeichnet, daß die Umsetzung mit einer zwei- bzw. dreistufigen Temperaturkaskade durchgeführt wird.

9. Verfahren nach einem der Ansprüche 1 bis 8, dadurch gekennzeichnet, daß die Temperatur im Bereich von 20 bis 90°C liegt.

10. Verfahren nach einem der Ansprüche 1 bis 9, dadurch gekennzeichnet, daß die Temperaturkaskadierung in einer dreistufigen Reaktorkaskade bei Temperaturen von 20-40°C/40-70°C/70-90°C, durchgeführt wird.

## Claims

1. A process for the preparation of a formate-containing polyalcohol product by reacting an alkanal or ketone with formaldehyde in aqueous solution in the presence of a tertiary amine, wherein the reaction is carried out using a temperature gradient with increasing temperature, and for 0.5-24 hours, and, based on 1 mol of alkanal or ketone, from 0.6 to 5 mol of tertiary amine are used and the process is carried out without hydrogenation of the product mixture.

2. A process for the preparation of a polyalcohol, which comprises the stages:
(a) reaction of an alkanal or ketone with formaldehyde in aqueous solution in the presence of a tertiary amine to give a formate-containing polyalcohol product mixture according to the process as claimed in claim 1,
(b) removal of water, excess tertiary amine and excess formaldehyde,
(c) heating of the remaining mixture from (b) with removal of further formaldehyde and tertiary amine and formation of formates of the polyalcohol,
(d) transfer of tertiary amine removed from stage (b) and/or from stage (c) to synthesis stage (a) and/or to the subsequent transesterification stage (e),
(e) transesterification of the resulting formates of the polyalcohol from stage (c) with an alcohol of the formula ROH in the presence of a transesterification catalyst to give polyalcohols and formates of the formula 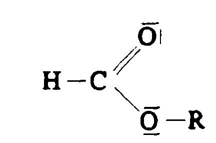 where R is a hydrocarbon radical, and
(f) isolation of polyalcohols.

3. A process as claimed in claim 2, wherein the transesterification catalyst is a tertiary amine.

4. A process as claimed in claim 2 or 3, wherein the transesterification is carried out in the presence of a trialkylamine as a transesterification catalyst, the amount of the trialkylamine used being 0.01-75 mol %, based on 1 mol of formate of the polyalcohol formate as 100 mol %.

5. A process as claimed in any of claims 2 to 4, wherein the material isolated from stages (c) and (b) is separated into the following mixtures in a stage (g):
1. an alcohol/tertiary amine/water mixture, at least some of which is fed to stage (e),
2. a mixture of water and formaldehyde, at least some of which is fed to stage (a), and
3. an aqueous solution enriched with medium boilers.

6. A process as claimed in any of claims 2 to 5, wherein the process is carried out without hydrogenation of the product mixture of stage (a).

7. A process as claimed in any of claims 1 to 6, wherein the ratio of the reactants is 0.5-10 mol of formaldehyde, based on 1 mol of alkanal or ketone.

8. A process as claimed in any of claims I to 7, wherein the reaction is carried out using a two- or three-stage temperature gradient.

9. A process as claimed in any of claims 1 to 8, wherein the temperature is from 20 to 90°C.

10. A process as claimed in any of claims 1 to 9, wherein the temperature gradation is effected in a three-stage reactor cascade at 20-40°C/40-70°C/70-90°C.

## Revendications

1. Procédé dé préparation d'un produit polyalcool contenant des formiates par réaction d'un alcanal ou d'une cétone avec du formaldéhyde en solution aqueuse en présence d'amine tertiaire, caractérisé en ce que la réaction est menée avec une cascade de températures, à température croissante, et pendant une durée de 0,5-24 heures, et en ce que l'on utilise 0,6-2,5 mole d'amine tertiaire pour 1 mole d'alcanal ou de cétone et en ce que le procédé est mené sans hydrogénation du mélange de produits.

2. Procédé de préparation de polyalcools, comprenant les étapes de :
(a) réaction d'un alcanal ou d'une cétone avec du formaldéhyde en solution aqueuse en présence d'une amine tertiaire en un mélange de produits polyalcools contenant des formiates selon le procédé de la revendication 1,
(b) séparation de l'eau, de l'amine tertiaire en excès, du formaldéhyde en excès,
(c) chauffage du mélange restant de (b) avec séparation du formaldéhyde et de l'amine tertiaire résiduels et formation de formiates de polyalcools,
(d) envoi de l'amine tertiaire séparée depuis l'étape (b) et/ou l'étape (c) dans l'étape de synthèse (a) et/ou dans l'étape suivante de transestérification (e),
(e) transestérification des formiates de polyalcools obtenus dans l'étape (c) avec un alcool de formule ROH en présence d'un catalyseur de transestérification en polyalcools et formiates de formule 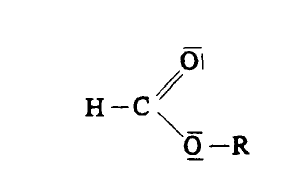 où R représente un reste hydrocarboné,
(f) récupération des polyalcools.

3. Procédé selon la revendication 2, caractérisé en ce que le catalyseur de transestérification est une amine tertiaire.

4. Procédé selon la revendication 2 ou 3, caractérisé en ce que la transestérification est menée en présence d'une trialkylamine en tant que catalyseur de transestérification, la quantité de la trialkylamine utilisée se trouvant dans la gamme de 0,01-75% en moles, par rapport à 1 mole de formiate du formiate de polyalcool en tant que 100% en moles.

5. Procédé selon l'une quelconque des revendications 2 à 4, caractérisé en ce que, dans une étape (g), le matériau séparé des étapes (c) et (b) est divisé en les mélanges suivants :
1. un mélange alcool/amine tertiaire/eau qui est conduit au moins en partie dans l'étape (e),
2. un mélange d'eau et de formaldéhyde qui est conduit au moins en partie dans l'étape (a), et
3. une solution aqueuse enrichie avec des produits à ébullition moyenne.

6. Procédé selon l'une quelconque des revendications 2 à 5, caractérisé en ce que le procédé est mené sans hydrogénation du mélange de produits de l'étape (a).

7. Procédé selon l'une quelconque des revendications 1 à 6, caractérisé en ce que le rapport des composants réactionnels, par rapport à 1 mole d'alcanal ou de cétone, se trouve dans la gamme de 0,5-10 moles de formaldéhyde.

8. Procédé selon l'une quelconque des revendications 1 à 7, caractérisé en ce que la réaction est menée avec une cascade de températures en deux ou trois étapes.

9. Procédé selon l'une quelconque des revendications 1 à 8, caractérisé en ce que la température se trouve dans la gamme de 20-90°C.

10. Procédé selon l'une quelconque des revendications 1 à 9, caractérisé en ce que la cascade de températures est mise en oeuvre dans un réacteur en cascade à trois étages, à des températures de 20-40°C / 40-70°C / 70-90°C.
